(19) Europäisches Patentamt
European Patent Office
Office européen des brevets

(11) Publication number: **0 033 760 A1**

# (12) EUROPEAN PATENT APPLICATION

(21) Application number: 80106315.7

(22) Date of filing: 16.10.80

(51) Int. Cl.³: C 07 C 41/03, B 01 J 23/02, B 01 J 31/00

(30) Priority: 04.02.80 US 118176

(43) Date of publication of application: 19.08.81 Bulletin 81/33

(84) Designated Contracting States: BE DE FR GB SE

(71) Applicant: CONOCO INC., 1000 South Pine Street P.O. Box 1267, Ponca City Oklahoma 74601 (US)

(72) Inventor: Kang, Yang, 2501 Robin Road, Ponca City, Oklahoma 74601 (US)
Inventor: Nield, Gerald Lee, 837 Edgewood, Ponca City, Oklahoma 74601 (US)
Inventor: Washecheck, Paul Howard, 2801 Canterbury, Ponca City, Oklahoma 74601 (US)

(74) Representative: Patentanwälte Dipl.-Ing. A. Grünecker, Dr.-Ing. H. Kinkeldey, Dr.-Ing. W. Stockmair,, Dr. rer. nat. K. Schumann, Dipl.-Ing. P.H. Jakob, Dr. rer. nat. G. Bezold Maximilianstrasse 43, D-8000 München 22 (DE)

## (54) A method for the alkoxylation of alkanols.

(57) Barium and strontium together with promoters are used as catalysts for the alkoxylation of alcohols of all classes. The reaction is carried out at temperatures of from about 90 °C to about 260 °C to yield an alkoxylation product. The products obtained have very narrow adduct distribution with low levels of by-products and unreacted free alcohols. The catalysts disclosed are preformed. These preformed catalysts reduce reaction time while providing narrow mole adduct distributions.

EP 0 033 760 A1

# A METHOD FOR THE ALKOXYLATION OF ALKANOLS

This invention relates to the production of alkoxylated alcohols by reacting said alcohols in the presence of barium-containing and strontium-containing catalysts and promoters. More particularly, this invention relates to the production of alkoxylated alcohols by reacting said alcohols in the presence of these barium-containing and strontium-containing catalysts which are preformed prior to reacting adducting materials such as ethylene oxide and propylene oxide.

The general reaction of alcohols and adducting materials such as ethylene oxide to form alkoxylated alcohols (ethylene oxide adducts) has long been known and practiced on a commercial scale. For example, ethylene oxide adducts have been used as detergents and cleaning agents, domestic and industrial laundry detergents, detergent builders, polishers, sanitizers, and dry-cleaning materials. Other users include the pulp and paper industry and the fiber industry. These materials are especially adapted to these uses since they have functional properties such as wetting power, foaming, emulsifying and dispersing abilities as well as solubilization and detergent abilities to facilitate their use.

Much literature is available in the general area of ethoxylation of alcohols. Many references are also available relating to the catalytic ability of various materials and the mechanism and kinetics of these reactions. For example, French Patent 1,365,945 teaches the use of compounds containing an active hydrogen atom reacted with ethylene oxide in the presence of an alkali metal base.

Acidic catalysts in general are also known. However, the ethoxylation of alcohols invariably produces a distribution of various adducts. For example on surfactant applications, an adduct of too few ethylene oxide molecules is not effective because of poor solubility. In contrast, an adduct with too many ethylene oxide molecules is likewise undesirable because of surface tension reduction per unit

mass decreases drastically with increase in the molecular weight. Thus it has long been essential to produce and use ethoxylates with as sharp a distribution in the desired mole adduct range (3 to 10 usually) as possible. Acid-catalyzed reactions produce such alkoxylates but these catalysts produce higher levels of some harmful side products (such as dioxane) which must be separated and removed prior to use.

Russian Patent 523,074 teaches that alkali metals and various carbonates can be used to catalyze these reactions. The side product formation in the base-catalyzed reactions is very low, but in base-catalyzed reactions the adduct distribution is undesirably broad. The result is that a large proportion of the product obtained is not useful or is less desirable because of distribution.

Representative of but not exhaustive of the art in this area is U.S. Patent 3,328,467 which describes the use of zeolites and modified zeolites as catalysts in ethoxylation reactions. French Patent 1,557,407 uses triethyl oxonium fluoroborate to catalyze such reactions. Indeed, the art abounds with references to alkali metal hydroxides such as sodium and potassium hydroxides, tertiary amines and sodium metal. German Offenlegungsschrift 2,639,564 teaches polyalkoxylation of active hydrogen compounds in the presence of a sodium fluoroborate or perchlorates of metal such as magnesium, calcium, manganese, or zinc. U.S. Patent 3,969,417 uses tertiary oxonium salts as a catalyst.

U.S. Patent 3,830,850 describes adding sodium, potassium, lithium, rubidium, cesium, calcium, barium, or strontium to condense phenols with formaldehyde, then adding ethylene oxide to the condensation product in an ethoxylation reaction. However, all these materials have the disadvantages described and set forth above.

Great benefit would be provided by a catalyst system which provides the low by-product levels of base catalysts yet has the narrow distribution of the preferred mole adducts obtained from acid catalysts together with reduced reaction time. Such a catalyst which would promote the narrowing of the product distribution curve would contribute significantly to the intrinsic value of the

ethoxylate produced. Such a catalyst is described in U.S. application Serial Number 916,421, filed June 6, 1978. However, this catalyst has an induction period ranging up to about 20 minutes at 178°C.

It is therefore an object of the present invention to provide a catalyst system which will yield a narrow mole adduct distribution from the reaction of alcohols of all classes with materials such as ethylene oxide and propylene oxide while providing low levels of undesirable by-products and unreacted free alcohols, yet provide a reaction which is immediately effective with reduced induction period. Other objects will become apparent to those skilled in this art as the description proceeds.

It has now been discovered according to the present invention that alkoxylation of alkanols containing from about 4 to about 36 carbon atoms can be carried out by contacting the alcohols with an alkoxylation agent in the presence of at least one material selected from the group consisting of barium metal, barium hydride, barium oxide, barium hydroxide, hydrated barium hydroxide, strontium metal, strontium hydride, strontium oxide, strontium hydroxide, hydrated strontium hydroxide, or mixtures of these together with an effective amount of a catalyst promoter or mixture of catalyst promoters, where the strontium and/or barium catalyst is preformed with a promoter, and wherein the alkoxylation reaction is carried out at temperatures of from about 120°C to about 260°C and wherein the catalyst promoter is selected from the group consisting of

a) polyols having a boiling point above 100°C and containing a total of 2 to 30 carbon atoms; and having 2 or more adjacent or non-adjacent hydroxyl containing groups of the general formula

$$R_1 - \overset{\displaystyle R_2}{\overset{|}{\underset{\underset{\displaystyle R_3}{|}}{C}}} - OH$$

wherein $R_1$, $R_2$, and $R_3$ are, independently, linear or branched acyclic groups, alicyclic groups, aryl

groups, cyclic groups, or hydrogen and wherein the R-designated groups can in addition contain one or more functional groups selected from the group consisting of ether, amine, carboxyl, halogen, nitro, carbonyl, and amide;

b) aldehydes and ketones having boiling points above 100°C and containing a total of from 2 to 30 carbon atoms, and having one or more adjacent or non-adjacent carbonyl containing groups of the general formula

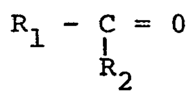

wherein $R_1$ and $R_2$ are, independently, hydrogen, linear or branched acyclic groups, alicyclic groups, cyclic groups, or aryl groups and wherein the R-designated groups can in addition contain one or more functionalities selected from the group consisting of carboxyl, hydroxyl, ether, halogen, nitro, amine, or amide;

c) primary, secondary or tertiary amides having a boiling point of above 100°C and containing a total of from 1 to 30 carbon atoms and containing 1 or more amide containing groups of the general formula

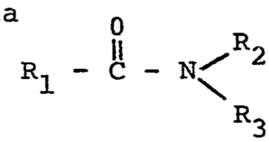

wherein $R_1$, $R_2$, and $R_3$ are, independently hydrogen, linear or branched acyclic groups, alicyclic groups, cyclic groups or aryl groups and wherein the R-designated groups can in addition contain one or more other functionalities selected from the group consisting of hydroxyl, ether, carboxyl, carbonyl, amine, nitro, or halogen;

d) primary, secondary or tertiary amines having a boiling point above 100°C, containing from a total of 1 to 30 carbon atoms and containing 1 or more amine containing groups of the general formula

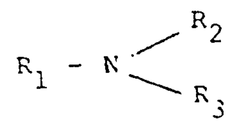

wherein $R_1$, $R_2$ and $R_3$ are, independently, hydrogen, linear or branched acyclic groups, alicyclic groups, cyclic groups, or aryl groups, and wherein the R-designated groups can in addition contain one or more functionalities selected from the group consisting of hydroxyl, ether, carbonyl, halogen, carboxyl, nitro or amide;

e) organic acids having a boiling point of above 100°C, containing from a total of 1 to 30 carbon atoms and having 1 or more adjacent or non-adjacent carboxylic acid containing groups of the general formula

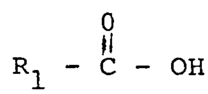

wherein $R_1$ is hydrogen, a linear or branched acyclic group, alicyclic group, cyclic group, or aryl group and wherein the R group can in addition contain one or more functionalities selected from the group consisting of carbonyl, hydroxyl, halogen, ether, nitro, amine, or amide;

f) phenols having a boiling point of above 100°C, containing a total of from 6 to 30 carbon atoms and having 1 or more functionalities of the general formula

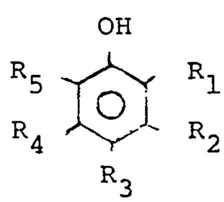

wherein $R_1$, $R_2$, $R_3$, $R_4$, and $R_5$ are, independently hydrogen, halogen, hydroxyl, nitro, ether, or carbonyl, linear or branched acyclic groups, alicyclic groups, cyclic groups, aryl groups, or substituted aryl groups and wherein in addition the R-designated groups can contain one or more

functionalities selected from the group consisting of halogen, ether, nitro, carboxyl, carbonyl, amine, amide, or hydroxyl. Boiling points are measured at atmospheric pressure, and may be proportionally higher or lower as pressure is altered.

These preformed catalysts are produced by simply contacting the catalytic metal compound and promoter together for a time and at a temperature sufficient to obtain the preformed material. Normally such reaction time ranges up to about two hours, but from 1/2 to one hour is preferred. Reaction temperatures range up to about 175°C, but reaction temperatures from about 100°C to about 150°C are preferred.

For handling ease, a solvent effective for either or both the catalyst and promoter can optionally be used. Such solvents can operably but not necessarily be removed by heating until the preformed catalyst is obtained as a solid. The preferred catalysts of the present invention can be obtained as liquids if desired. Heating the preformed catalyst for a time and at a temperature sufficient to remove bound water is preferred, but is not critical. In many cases, the solvent is a material in itself capable of promoting the alkoxylation reaction and of producing a preformed catalyst.

Representative examples of various polyol promoters which are effective in the process of the instant invention as a preformed catalyst are:

ethylene glycol
1,2-propylene glycol
1,4-butanediol
1,6-hexanediol
1,10-decanediol
1,3-butylene glycol
diethylene glycol
diethylene glycol monobutyl ether
diethylene glycol monomethyl ether
diethyl glycol monoethyl ether
dipropylene glycol
dipropylene glycol monomethyl ether
ethylene glycol monomethyl ether
ethylene glycol monoethyl ether
ethylene glycol monobutyl ether
hexylene glycol

0033760



mannitol
sorbitol
pentaerythritol
dipentaerythritol
tripentaerythritol
trimethylolpropane
trimethylolethane
neopentyl glycol
diethanolamine
triethanolamine
diisopropanolamine
triisopropanolamine
1,4-dimethylolcyclohexane
2,2-bis(hydroxymethyl)propionic acid
1,2-bis(hydroxymethyl)benzene
4,5-bis(hydroxymethyl)furfural
4,8-bis(hydroxymethyl)tricyclo[5,2,1,0] decane
tartaric acid
2-ethyl-1,3-hexanediol
2-amino-2-ethyl-1,3-propanediol
triethylene glycol
tetraethylene glycol
glycerol
ascorbic acid

Representative examples of various aldehydes and ketones which are effective in the process of the present invention as preformed catalysts are:

lauryl aldehyde
benzaldehyde
2-undecanone
acetophenone
2,4-pentandione
acetylsalicylic acid
ortho-chlorobenzaldehyde
para-chlorobenzaldehyde
cinnamic aldehyde
diisobutyl ketone
ethylacetoacetate
ethyl amyl ketone
camphor
para-hydroxybenzaldehyde
2-carboxybenzaldehyde
4-carboxybenzaldehyde
salicylaldehyde
octyl aldehyde
decyl aldehyde
p-methoxybenzaldehyde
p-aminobenzaldehyde
phenylacetaldehyde
acetoacetic acid
2,5-dimethoxybenzaldehyde
1-naphthyl aldehyde
terephthaldehyde

Representative examples of amides which are effective promoters in the process of the instant invention as preformed catalysts are:

formamide
benzamide
acetanilide
salicylamide
acetoacetanilide
ortho-acetoacetotoluidide
acrylamide
N,N-diethyltoluamide
N,N-dimethylacetamide
N,N-dimethylformamide
phthalimide
octylamide
decylamide
laurylamide
stearylamide
N,N-dimethylollaurylamide
N,N-dimethylacrylamide
para-chlorobenzamide
para-methoxybenzamide
para-aminobenzamide
para-hydroxybenzamide
ortho-nitrobenzamide
N-acetyl-para-aminophenol
2-chloroacetamide
oxamide
N,N-methylene-bis-acrylamide

Representative examples of amines which are effective promoters in the process of the present invention as preformed catalysts are:

aniline
benzylamine
hexadecylamine
triphenylamine
aminoacetic acid
anthranilic acid
cyclohexylamine
tert-octylamine
ortho-phenylenediamine
meta-phenylenediamine
para-phenylenediamine
N-acetyl-para-aminophenol
2-amino-4-chlorophenol
2-amino-2-ethyl-1,3-propanediol
ortho-aminophenol
para-aminophenol
para-aminosalicylic acid
benzyl-N,N-dimethylamine
tert-butylamine
2-chloro-4-aminotoluene

6-chloro-2-aminotoluene
meta-chloroaniline
ortho-chloroaniline
para-chloroaniline
4-chloro-2-nitroaniline
cyclohexylamine
dibutylamine
2,5-dichloroaniline
3,4-dichloroaniline
dicyclohexylamine
diethanolamine
N,N-diethylethanolamine
N,N-diethyl-meta-toluidine
N,N-diethylaniline
diethylenetriamine
diisopropanolamine
N,N-dimethylethanolamine
N,N-dimethylaniline
2,4-dinitroaniline
diphenylamine
ethyl-para-aminobenzoate
N-ethylethanolamine
N-ethyl-1-naphthylamine
N-ethyl-ortho-toluidine
N-ethylaniline
ethylenediamine
hexamethylenetetraamine
2,4-lutidine
N-methylaniline
methyl anthranilate
p,p'-diaminodiphenyl methane
ortho-nitroaniline
para-nitroaniline
tert-octylamine
piperazine
ethanolamine
isopropanolamine
ortho-toluidine
para-toluidine
2,4-tolyenediamine
triethanolamine
tributylamine
triisopropanolamine
2,4-dimethylxylidine
para-methoxyaniline
nitrilotriacetic acid
N-phenyl-1-naphthylamine

Representative examples of acids which are effective promoters in the process of the present invention as preformed catalysts are:

formic acid
acetic acid
valeric acid
heptanoic acid

2-ethylhexanoic acid
lauric acid
stearic acid
oleic acid
tall oil acids
hydrogenated tall oil acids
benzoic acid
salicylic acid
adipic acid
azelaic acid
fumaric acid
citric acid
acrylic acid
aminoacetic acid
para-aminosalicylic acid
anthranilic acid
butyric acid
propionic acid
ricinoleic acid
chloroacetic acid
ortho-chlorobenzoic acid
2,4-dichlorophenoxyacetic acid
tert-decanoic acid
para-aminobenzoic acid
abietic acid
itaconic acid
lactic acid
glycolic acid
malic acid
maleic acid
cinnamic acid
para-hydroxybenzoic acid
methacrylic acid
oxalic acid
myristic acid
palmitic acid
tert-pentanoic acid
phenylacetic acid
mandelic acid
sebacic acid
tallow fatty acids
hydrogenated tallow fatty acids
tartaric acid
trichloroacetic acid
2,4,5-trichlorophenoxyacetic acid
undecylenic acid
crotonic acid
pelargonic acid
acetoacetic acid
para-nitrobenzoic acid
ascorbic acid
nitrilotriacetic acid
naphthenic acids
1-naphthoic acid
trimellitic acid

Representative examples of various phenols which are promoters for the preformed catalysts of the present invention are:

phenol
ortho-cresol
meta-cresol
para-cresol
2,4-dimethylphenol
2,5-dimethylphenol
2,6-dimethylphenol
ortho-chlorophenol
meta-chlorophenol
para-chlorophenol
para-nitrophenol
para-methoxyphenol
salicylic acid
meta-hydroxyacetophenone
para-aminophenol
ortho-phenylphenol
nonylphenol
octylphenol
t-butyl-para-cresol
hydroquinone
catechol
resorcinol
pyrogallol
1-naphthol
4,4'-isopropylidenediphenol (bisphenol A)
methyl salicylate
benzyl salicylate
4-chloro-2-nitrophenol
para-t-butylphenol
2,4-di-t-amylphenol
2,4-dinitrophenol
para-hydroxybenzoic acid
8-hydroxyquinoline
methyl para-hydroxybenzoate
2-nitro-para-cresol
ortho-nitrophenol
para-phenylphenol
phenyl salicylate
salicylaldehyde
p-hydroxy benzaldehyde
2-amino-4-chlorophenol
ortho-aminophenol
salicylamide
2,4-dichlorophenol
2,5-dichlorophenol
2,5-dichlorohydroquinone
2-naphthol

Certain alcohols can also act as promoters for ethoxylation of alcohols using barium-containing and strontium-containing catalysts. Such alcohols are more acidic than normal alcohols such that the association constant is greater

(or the pKa is lower) than normal alcohols. Thus, these alcohol promoters would have a pKa less than 1.

These alcohols are those having the general formula

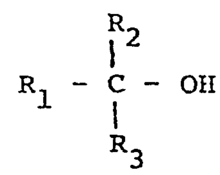

wherein $R_1$, $R_2$ and $R_3$ are, independently, hydrogen, linear or branched acyclic groups, alicyclic groups, aryl groups, or cyclic groups and wherein the R-designated groups can in addition contain functional groups selected from the group consisting of halogen, nitro, carboxyl, amine, carbonyl, ether, and amide. The molecule can contain a total of from 2 to 30 carbon atoms.

Representative examples of these effective alcohols which can be used to preform catalysts are:

triphenylmethanol
trichloroethanol
trifluoroethanol
2-nitroethanol
2-chloroethanol
2,2-dichloroethanol
2-methoxyethanol
2-chlorocyclohexanol
ortho-chlorobenzyl alcohol

Thus, the instant invention describes a method for the alkoxylation of alcohols comprising contacting said alcohols with an alkoxylating agent in the presence of strontium-containing and barium-containing catalysts together with an effective amount of a polyol, aldehyde, ketone, amine, amide, carboxylic acid, alcohol, substituted phenol or mixtures of these preformed into a catalyst effective to promote the reaction. Normally, the alkoxylation agent will be ethylene oxide or propylene oxide. The instant invention can be carried out at temperatures of from about 90°C to about 260°C. Normally, the alcohols reactive under the process of the instant invention will contain from about 4 to about 36 carbon atoms but alcohols of from about 4 to about 24 carbon atoms are more common. Those alcohols containing from about 8 to about 18 carbon atoms are most used for commercial processes.

The process of the instant invention can be carried out at ambient pressures. However, pressures of up to 100 pounds per square inch gauge (psig) can also be used. Pressures below about 60 psig are preferred. In addition, pressures below ambient can be used. It is clear that while pressure or lack of pressure is not a detriment to the process of the instant invention, it is simply more convenient to carry out the reaction in the pressure range of from about atmospheric to about 100 psig.

The instant invention is normally carried out at temperatures of from about 120 to about 260°C. However, for practical purposes, commercial operations will normally be carried out in the temperature range of from about 150 to about 200°C. Temperatures in the range of from 160°C to about 190°C are most preferred.

Reactions can be carried out in the presence of any alkoxylation agent which produces a mole adduct of the sort desired. Normally such agents are alpha or beta alkylene oxides. In most commercial operations, either ethylene oxide, propylene oxide, or mixtures of these will be used to produce an adduct. Of these products, ethylene oxide is most preferred.

Reaction products can have any desired content of alkoxylating agent such as ethylene oxide, but will normally range from about 30 to about 80% content of ethylene oxide (EO) based on weight. However, for most purposes, the content of ethylene oxide will range from about 40% to about 70% by weight. The amount of EO present in the reaction is not critical other than the minimum amount necessary to provide sufficient units to reach the mole adduct level desired for the alcohol being reacted.

The catalysts of the present invention are basic catalysts which provide a sharp distribution of mole adducts formed, while greatly reducing the amount of unreacted free alcohols and undesirable by-products normally found in sharp distribution reactions. The instant invention adds to this barium-containing and strontium-containing catalysts an effective amount of phenol, acid, amine, aldehyde, polyol, ketone, amide or alcohol and preforms the metal compound and

promoter in order to further reduce by-product reactions and to reduce or eliminate the induction period necessary for alkoxylation to begin.

Representative examples of barium-containing catalysts are barium metal, $BaH_2$, BaO, $Ba(OH)_2$ and $Ba(OH)_2.X H_2O$ where X represents the number of water molecules present. X is not a critical number. Many of these barium compounds alone are effective in the process of the instant invention and are extremely active when used as a preformed catalyst with an effective amount of a promoter.

Representative examples of strontium-containing catalysts are strontium metal, $SrH_2$, SrO, $Sr(OH)_2$ and $Sr(OH)_2.X H_2O$ where X represents the number of water molecules present. X is not a critical number. Many of these strontium compounds are effective alone in the process of the instant invention and are extremely effective when used as a preformed catalyst with an effective amount of a promoter.

Barium and strontium catalysts in their various forms and with the promoters listed can be used together with excellent results. Many combinations are possible and while some will be more effective than others, all will beneficially promote alkoxylation of alcohols. Synergistic results can be expected with mixtures of metal catalysts and/or promoters.

When used, these catalyst mixtures can be used in any desired quantity. The larger the quantity used, the more quickly the reaction goes to completion, although larger quantities do not appear to significantly alter the distribution obtained. However, for practical purposes, normally from about 0.1 weight percent preformed catalyst based upon the weight of the alcohol to be reacted, would be present in the reaction, but from 0.1 to 5.0 weight percent is more commonly used, and from 0.1 to about 2.0 weight percent is most preferred. The amount of promoter or co-catalyst which should be present in the preformed catalyst is generally an effective amount. The effect of the co-catalyst or promoter becomes significant at about 0.1% by weight based upon the weight of the alcohol to be reacted. It is logical to expect an upper limit after which the amount of promoter present will produce no additional benefits, especially

since preforming the catalyst further reduces induction periods, even to insignificant levels.

Normally, these materials will be added to barium and strontium compounds in amounts ranging from about 0.1 to about 2.0% by weight based upon the weight of the alcohol to be reacted, then preformed. Although amounts ranging from about 0.15 to about 1.5 are preferred, and amounts ranging from about 0.3 to about 0.8% by weight based upon the weight of alcohol to be reacted is most preferred. However, it is very apparent that these limits can be varied substantially since the co-catalyst is more effective when preformed.

While the instant invention is effective with all classes of alkanols, both primary, secondary, tertiary, linear and branched, linear and branched primary alkanols are the most commonly used alcohols and are the preferred alcohols of the instant invention. Representative examples of such alcohols are those derived by hydrogenation of natural fats and oils, such as CO and TA alcohols, trademark of and sold by Proctor and Gamble Co., such as CO 1214N alcohol, CO 1618 alcohol, and TA 1618 alcohol, and ADOL alcohols, trademark of and sold by Ashland Oil Co., such as ADOL 54 alcohol, ADOL 61 alcohol, ADOL 64 alcohol, ADOL 60 alcohol and ADOL 66 alcohol. Alcohols produced by Ziegler chemistry can also be ethoxylated. Examples of these alcohols are ALFOL alcohols, trademark of and sold by Conoco Inc, such as ALFOL 1012 alcohol, ALFOL 1214 alcohol, ALFOL 1412 alcohol, ALFOL 1618 alcohol, ALFOL 1620 alcohol; and EPAL alcohols, trademark of and sold by Ethyl Chemical Co., such as EPAL 1012 alcohol, EPAL 1214 alcohol, EPAL 1418 alcohol. The invention is extremely useful for oxo alcohols (hydroformylation) produced from olefins. Examples of such alcohols are NEODOL alcohol, trademark of and sold by Shell Oil Co., such as NEODOL 23 alcohol, NEODOL 25 alcohol, NEODOL 45 alcohol; TERGITOL-L, trademark of Union Carbide Corp., such as TERGITOL-L 125 alcohol; LIAL alcohols, trademark of and sold by Liquichimica Co. such as LIAL 125; and isodecyl and tridecyl alcohols, sold by Exxon Corp., such as isodecyl alcohol and tridecyl alcohol. Guebert alcohols can also be ethoxylated. Representative examples of these alcohols are STANDAMUL alcohols,

trademark of and sold by Henkel Chemical Co., such as STANDAMUL GT-12 alcohol, STANDAMUL GT-16 alcohol, STANDAMUL GT-20 alcohol, STANDAMUL GT-1620 alcohol. Secondary alcohols can also be used, such as TERGITOL 15 alcohol, trademark of and sold by Union Carbide Corp.

Representative examples of such alcohols are 1-decanol; 1-undecanol; 1-dodecanol; 1-tridecanol; 1-tetradecanol; 1-pentadecanol; 1-hexadecanol; 1-heptadecanol; 1-octadecanol; 1-nonadecanol; 1-eicosanol; 1-docosanol; 2-methyl-1-undecanol; 2-propyl-1-nonanol; 2-butyl-1-octanol; 2-methyl-1-tridecanol; 2-ethyl-1-dodecanol; 2-propyl-1-undecanol; 2-butyl-1-decanol; 2-pentyl-1-nonanol; 2-hexyl-1-octanol; 2-methyl-1-pentadecanol; 2-ethyl-1-tetradecanol; 2-propyl-1-tridecanol; 2-butyl-1-dodecanol; 2-pentyl-1-undecanol; 2-hexyl-1-decanol; 2-heptyl-1-decanol; 2-hexyl-1-nonanol; 2-octyl-1-octanol; 2-methyl-1-heptadecanol; 2-ethyl-1-hexadecanol; 2-propyl-1-pentadecanol; 2-butyl-1-tetradecanol; 1-pentyl-1-tridecanol; 2-hexyl-1-dodecanol; 2-octyl-1-decanol; 2-nonyl-1-nonanol; 2-dodecanol; 3-dodecanol; 4-dodecanol; 5-dodecanol; 6-dodecanol; 2-tetradecanol; 3-tetradecanol; 4-tetradecanol; 5-tetradecanol; 6-tetradecanol; 7-tetradecanol; 2-hexadecanol; 3-hexadecanol; 4-hexadecanol; 5-hexadecanol; 6-hexadecanol; 7-hexadecanol; 8-hexadecanol; 2-octadecanol; 3-octadecanol; 4-octadecanol; 5-octadecanol; 6-octadecanol; 7-octadecanol; 8-octadecanol; 9-octadecanol; 9-octadecenol-1; 2,4,6-trimethyl-1-heptanol; 2,4,6,8-tetramethyl-1-nonanol; 3,5,5-trimethyl-1-hexanol; 3,5,5,7,7-pentamethyl-1-octanol; 3-butyl-1-nonanol; 3-butyl-1-undecanol; 3-hexyl-1-undecanol; 3-hexyl-1-tridecanol; 3-octyl-1-tridecanol; 2-methyl-2-undecanol; 3-methyl-3-undecanol; 4-methyl-4-undecanol; 2-methyl-2-tridecanol; 3-methyl-3-tridecanol; 4-methyl-3-tridecanol; 4-methyl-4-tridecanol; 3-ethyl-3-decanol; 3-ethyl-3-dodecanol; 2,4,6,8-tetramethyl-2-nonanol; 2-methyl-3-undecanol; 2-methyl-4-undecanol; 4-methyl-2-undecanol; 5-methyl-2-undecanol; 4-ethyl-2-decanol; 4-ethyl-3-decanol; tetracosanol; hexacosanol; octacosanol; triacontanol; dotriacontanol; hexatriacontanol; 2-decyltetradecanol; 2-dodecylhexadecanol; 2-tetradecyloctadecanol; 2-hexadecyleicosanol.

Generally, the treatment of alcohols with ethylene oxide yields a non-ionic detergent since hydrogen bonding to the numerous oxygen atoms makes the polyether end of the molecule water soluble. Alternatively, the ethoxylates can be converted into sulfates and used in the form of alkali metal or ammonium salts.

The instant invention provides for the production of highly efficient alcohol alkoxylates from primary, secondary and tertiary branched chain and straight chained alcohols in a novel, highly unexpected manner. Of these, primary alkanols are preferred. These alkanols normally have from about 4 to about 20 carbon atoms, but from about 4 to about 36 carbon atoms can also be used. Reaction products are useful as non-ionic surface active agents with high wetting power and are composed with mixtures of mono-alkyl ethers of polyethylene glycol.

Thus in the preferred form of the instant invention, ethylene oxide is reacted with a branched chain or straight chain higher alkanol in the presence of barium bases or strontium bases promoted by an effective amount of amines, amides, aldehydes, polyols, carboxylic acids, ketones, and substituted phenols which have been pre-formed into an alkoxylation catalyst.

The invention is more concretely described with reference to the examples below wherein all parts and percentages are by weight unless otherwise specified. The examples are provided to illustrate the instant invention and not to limit it.

Example 1

A small round bottom flask was charged with 10 grams $Sr(OH)_2 \cdot 8\ H_2O$ and 10.6 grams phenol. The flask was attached to a rotary evaporator and the contents heated to 150°C for 1 hour under vacuum. The weight of 9.55 grams of preformed catalyst was recovered.

Example 2

A stainless steel reactor (600 cc) was charged with 150 grams Alfol 1214 alcohol (trademark of and sold by Conoco Inc.) and 0.8 grams preformed strontium hydroxide/ phenol catalysts as prepared in Example 1. After purging

with nitrogen at 250 cc per minute for 1 hour at a temperature of 150°C the reactor was evacuated and the temperature raised to about 175°C. Ethylene oxide (EO) was then introduced to a total pressure of about 40 pounds per square inch gauge (psig) and an EO uptake of 150 grams was allowed to proceed at this pressure. After ethoxylation the catalyst was neutralized. Ethoxylation required 65 minutes for completion. The product contained 0.5% polyethylene glygol (PEG) and had a pour point of 40 °F. Free alcohol content in the product was 3.8%.

Example 3

A small flask was charged with 10.0 grams $Sr(OH)_2.H_2O$ and 10.7 grams 1-naphthol together with 17.8 grams of ethanol. The mixture was stirred for 30 minutes at about 24°C and the solution filtered through Whatman No. 50 filter paper. The resulting filtrate was a dark fluid preformed catalyst easily handled in commercial operations.

Example 4

A stainless steel reactor of 600 cc capacity was charged with 120 grams of Alfol 1214 alcohol and 1.6 grams of preformed catalyst described in Example 3. After purging with nitrogen at 250 cc per minute for 1 hour at a temperature of 150°C, the reactor was evacuated and the temperature raised to about 175°C. Ethylene oxide was then introduced to a total pressure of about 40 psig and an EO uptake of 180 grams was allowed to proceed at this pressure. After ethoxylation, the catalyst was neutralized. The ethoxylation required 141 minutes for completion.

Example 5

A 250 cc flask was charged with 10 grams of $Sr(OH)_2.H_2O$, 7.8 grams of phenol, and 10.0 grams of water. The mixture was stirred at about 60°C and the resulting solution filtered through Whatman No. 50 filter paper. The resulting preformed catalyst filtrate was a light colored fluid.

Example 6

An experiment was carried out as described in Example 2 except that Neodol 25 alcohol (trademark of and sold by Shell Chemical Company, a product of a hydroformylation/hydrogenation reaction), and 2.0 grams preformed catalyst as prepared in Example 5. Ethylene oxide addition was complete in 66 minutes. The product contained 0.5% PEG and had a pour point of 40°F.

Example 7

An experiment was carried out as described in Example 2 except that Neodol 25 alcohol and 1.9 grams of preformed catalyst prepared from 5.0 grams $Sr(OH)_2 \cdot 8 H_2O$ and 8.6 grams bisphenol A was used. The catalyst system was preformed by heating the reactants to 60°C while stirring in the presence of 25 grams of Formula 30 alcohol (Formula 30 alcohol is 90% ethanol and 10% methanol and is described in The Merck Index of Chemicals and Drugs, seventh edition, published by Merck and Co., Inc. (1960). The volatiles were removed from the catalyst preparation by applying heat and vacuum on a rotary evaporator. The resulting preformed catalyst weighed 11.1 grams. EO addition for ethoxylation required only 70 minutes compared to 250 minutes for non-preformed catalysts as described in Example 8. The relative reaction rates are shown in Figure 1.

Example 8

An experiment was carried out as described in Example 7 except that the catalyst was not preformed. Strontium hydroxide.$8H_2O$ and bisphenol A (0.8 grams and 1.4 grams respectively) were each added to the reaction system as separate co-catalysts. EO addition required 250 minutes. The comparison in reaction time is set forth graphically in Figure 1.

Example 9

An experiment was carried out as described in Example 2 except that Neodol 25 alcohol and 0.85 grams of preformed catalyst was used. The catalyst system was preformed by heating 5.0 grams $Sr(OH)_2 \cdot 8H_2O$ and 3.54 grams azelaic acid in the presence of Formula 30 alcohol.

Volatiles were subsequently removed by applying heat and vacuum on a rotary evaporator. A weight of 5.3 grams of preformed catalyst was obtained from the reaction. EO addition for the ethoxylation required 135 minutes as compared to 288 minutes for a nonpreformed catalyst as described in Example 10. Relative reaction rates are set forth graphically in Figure 2.

Example 10

An experiment was carried out as described in Example 9 except that the catalyst was not preformed. Catalyst components were added as 0.8 grams $Sr(OH)_2.8H_2O$ and 0.56 grams azelaic acid. EO addition required 288 minutes. The reaction is set forth graphically in Figure 2.

Example 11

An experiment was carried out as described in Example 2 except that Neodol 25 alcohol and .77 grams of preformed catalyst was used. The catalyst was prepared from 5.0 grams $Sr(OH)_2.8H_2O$ and 2.69 grams salicylic acid. The catalyst system was preformed by heating the reactions in the presence of Formula 30 alcohol and subsequently removing the volatiles by applying heat and vacuum on a rotary evaporator. A rate of 4.8 grams of preformed catalyst was obtained. EO addition for the ethoxylation required 233 minutes as compared to 376 minutes with a non-preformed catalyst. Relative reaction rates are set forth graphically in Figure 3.

Example 12

An experiment was carried out as described in Example 11 except that the catalyst was not preformed. Strontium and salicylic acid components were added separately as catalyst and promoter. A weight of 0.8 grams of the strontium catalyst and 0.9 grams salicylic acid promoter were added separately to the system. EO addition required 376 minutes and is set forth in a comparative graphic example in Figure 3.

Example 13

An experiment was carried out as described in Example 2 except that Neodol 25 alcohol and 1.15 grams of preformed catalyst were used. The catalyst was prepared

from 5.2 grams $Sr(OH)_2.8H_2O$ and 4.29 grams of hydroquinone. The catalyst was preformed by heating the reactants in the presence of water and Formula 30 alcohol and subsequently removing volatiles by applying heat and vacuum on a rotary evaporator. A weight of 7.2 grams of preformed catalyst was obtained. EO addition for the ethoxylation required 110 minutes as compared to 173 minutes with a non-preformed catalyst. Relative reaction rates are set forth graphically in Figure 4.

Example 14

An experiment was carried out exactly as described in Example 13 except the catalyst was not preformed. A weight of 0.8 grams $Sr(OH)_2.8H_2O$ and 0.7 grams hydroquinone were each added separately as catalyst and promoter. Ethylene oxide addition required 173 minutes. The reaction is set forth graphically in Figure 4.

Example 15

An experiment was carried out as described in Example 2 except that Neodol 25 alcohol and 0.52 grams of preformed catalyst were used. The catalyst was preformed by heating 5.0 grams $Sr(OH)_2.8H_2O$ and 0.94 grams citric acid in the presence of water and Formula 30 alcohols. Volatiles were removed by applying heat and vacuum on a rotary evaporator. A weight of 3.3 grams of preformed catalyst was obtained. EO addition for the ethoxylation required 157 minutes as compared to 258 minutes when only 0.8 grams $Sr(OH)_2.8H_2O$ was used as a catalyst. The relative reaction rates are set forth graphically in Figure 5.

Example 16

A 250 cc flask equipped with a magnetic stirrer and reflux condensor was charged with 50 grams of Formula 30 alcohol and 6.4 grams benzoic acid. The mixture was stirred at ambient temperature and 5.0 grams $Ba(OH)_2.H_2O$ were slowly added. The mixture was heated and refluxed overnight. Volatiles were removed on a rotary evaporator and 7.1 grams of solid preformed barium catalyst was recovered.

Example 17

An experiment was carried out as described in Example 1 except that 6.9 grams heptanoic acid and 5.0 grams

$Ba(OH)_2.H_2O$ were used. The solid preformed catalyst recovered was 10.1 grams.

Example 18

An experiment was carried out as described in Example 1 except 2.9 grams hydroquinone and 5.0 grams $Ba(OH)_2.H_2O$ were used. The solid preformed catalyst recovered weighed 7.4 grams.

Example 19

An experiment was carried out as described in Example 1 except that 3.6 grams salicylic acid and 5.0 grams $Ba(OH)_2.H_2O$ were used. The solid preformed catalyst recovered weighed 7.0 grams.

While certain embodiments and details have been shown for the purpose of illustrating this invention, it will be apparent to those skilled in this art that various changes and modifications may be made herein without departing from the spirit or scope of the invention.

We claim:

## CLAIMS

1. A method for the alkoxylation of alkanols containing from about 2 to about 36 carbon atoms, comprising carrying out said alkoxylation in the presence of basic barium catalysts, strontium catalysts, and mixtures of these, said catalyst combined with an effective amount of a catalyst promoter or mixture of catalyst promoters prior to said reaction, said combining carried out at a temperature of up to about 175°C, and wherein the catalyst promoter is selected from the group consisting of

a) polyols containing a total of from 2 to 30 carbon atoms and having a boiling point above 100°C;

b) aldehydes and ketones containing a total of from 2 to 30 carbon atoms and having a boiling point above 100°C;

c) amides containing a total of from 1 to 30 carbon atoms and having a boiling point above 100°C;

d) amines containing a total of from 1 to 30 carbon atoms and having a boiling point above 100°C;

e) organic acids containing a total of from 1 to 30 carbon atoms and having a boiling point of 100°C;

f) phenols containing a total of from 6 to 30 carbon atoms and having a boiling point above 100°C;

2. A method as described in claim 1 wherein

a) the polyol promoters contain 2 or more adjacent or non-adjacent hydroxyl containing groups of the general formula

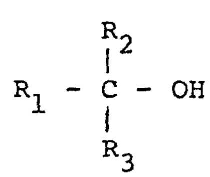

wherein $R_1$, $R_2$, and $R_3$ are, independently, linear or branched acyclic groups, alicyclic groups, cyclic groups, aryl groups or hydrogen and wherein the R-designated groups in addition can contain one or more functional groups selected from the group consisting of ether, amine, carboxyl, halogen, nitro, carbonyl, and amide;

b) the aldehyde and ketone promoters contain one or more adjacent or non-adjacent carbonyl containing groups of the general formula

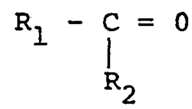

wherein $R_1$, and $R_2$ are, independently, linear or branched acyclic groups, alicyclic groups, cyclic groups, aryl groups or hydrogen, and wherein the R-designated groups in addition can contain one or more functional groups selected from the group consisting of hydroxyl, ether, carboxyl, carbonyl, amine, nitro or halogen;

c) the primary, secondary or tertiary amide promoters contain one or more amide containing groups of the general formula

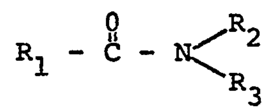

wherein $R_1$, $R_2$, and $R_3$ are, independently, hydrogen, linear or branched acyclic groups, alicyclic groups, cyclic groups or aryl groups and wherein the R-designated groups can in addition contain one or more functional groups selected from the group consisting of hydroxyl, ether, carboxyl, carbonyl, amine, nitro or halogen;

d) the primary, secondary or tertiary amine promoters contain one or more amine containing groups of the general formula

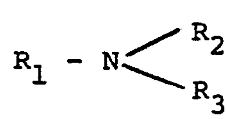

wherein $R_1$, $R_2$, and $R_3$, are, independently, hydrogen, linear or branched acyclic groups, alicyclic groups, cyclic groups, or aryl groups, and wherein the R-designated groups can in addition contain one or more functional groups selected from the group consisting of hydroxyl, ether, carbonyl, halogen, carboxyl, nitro, or amide;

e) the organic acid promoters contain one or more adjacent or non-adjacent carboxylic acid containing groups of the general formula

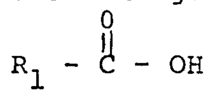

wherein $R_1$ is a hydrogen, linear or branched acyclic group, alicyclic group, cyclic group, or aryl group and wherein R can in addition, contain one or more functional groups selected from the group consisting of carbonyl, hydroxyl, halogen, ether, nitro, amine, or amide;

f) the phenol promoters have one or more functional groups of the general formula

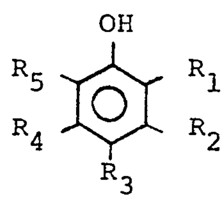

wherein $R_1$, $R_2$, $R_3$, $R_4$, and $R_5$ are, independently, hydrogen, halogen, hydroxyl, nitro, ether, or carbonyl, linear or branched acyclic groups, alicyclic groups, cyclic groups, aryl groups or substituted aryl groups wherein in addition the R-designated groups can contain one or more functional groups selected from the group consisting of halogen, ether, nitro, carboxyl, carbonyl, amine, amide, or hydroxyl; and wherein the barium and strontium compounds are selected from the group consisting of strontium metal, $SrH_2$, $SrO$, $Sr(OH)_2$, $Sr(OH)_2.X H_2O$, barium metal, $BaH_2$, $BaO$, $Ba(OH)_2$, or $Ba(OH)_2.XH_2O$.

3. A method as described in claim 2 wherein the alkoxylation is carried out using ethylene oxide, propylene oxide, or mixtures of these.

4. A method as described in claim 3 wherein the catalyst is selected from the group consisting of barium metal, barium hydride, barium hydroxide, hydrated barium

hydroxide, barium oxide, strontium metal, strontium hydride, strontium hydroxide, hydrated strontium hydroxide, or strontium oxide.

5. A method as described in claim 4 wherein the reaction is carried out at a pressure of up to about 100 pounds per square inch gauge.

6. A method as described in claim 5 wherein ethylene oxide is used as the alkoxylation agent and a mole adduct ratio ranges from about 30 weight percent to about 80 weight percent of the ethoxylated product.

7. A method as described in claim 6 wherein the barium or strontium containing catalyst or a mixture thereof is present in an amount of at least 0.1% by weight based upon the alcohol to be reacted.

8. A method as described in claim 7 wherein the alcohols to be ethoxylated contain from about 4 to about 20 carbon atoms.

9. A method as described in claim 8 wherein the alcohol is a primary alcohol.

10. A method as described in claim 8 wherein the alcohol is the product of a hydroformylation/hydrogenation reaction.

11. A method as described in claim 7 wherein the catalyst promoter is selected from the group consisting of heptanoic acid, ethylene glycol, benzoic acid, benzamide, adipic acid, phenol, stearic acid, bisphenol A (4,4'isopropylidene diphenol), triphenylmethanol, trimethylphenol, hexadecylamine, 1-naphthol, 2-naphthol, azelaic acid, fumaric acid, hydroquinone, salicylic acid, acetophenone, aniline, lauryl aldehyde, benzaldehyde, nonyl phenol, o-phenylphenol, benzylamine, citric acid, glycerine, acetylacetone, p-chlorophenol, formamide, 4-methyl-2-pentanone, pyridine, 1,4-butanedicl, propylene glycol, p-methoxyphenol, p-nitrophenol,

2-undecanone, 2,2,2-trifluoroethanol, acetic acid, mixtures of 2-ethylhexanoic acid and benzamide, 2-ethylhexanoic acid or 2-methoxyethanol.

12. A method as described in claim 11 when carried out in a continuous reaction.

13. A method of preparing an alkoxylation catalyst for the alkoxylation of alkanols containing from 2 to 36 carbon atoms with an alkoxylating agent selected from the group consisting of ethylene oxide, propylene oxide and butylene oxide, said catalyst comprising a metal compound selected from the group consisting of strontium metal, strontium hydride, strontium oxide, strontium hydroxide, hydrated strontium hydroxide, barium metal, barium hydride, barium oxide, barium hydroxide and hydrated barium hydroxide preformed with a promoter selected from the group consisting of

a) the polyol promoters contain 2 or more adjacent or non-adjacent hydroxyl containing groups of the general formula

```
        R2
        |
R1  -   C  -  OH
        |
        R3
```

wherein $R_1$, $R_2$, and $R_3$ are, independently, linear or branched acyclic groups, alicyclic groups, cyclic groups, aryl groups or hydrogen and wherein the R-designated groups in addition can contain one or more functional groups selected from the group consisting of ether, amine, carboxyl, halogen, nitro, carbonyl, and amide;

b) the aldehyde and ketone promoters contain one or more adjacent or non-adjacent carbonyl containing groups of the general formula

```
R1  -  C  =  O
       |
       R2
```

wherein $R_1$, and $R_2$ are, independently, linear or branched acyclic groups, alicyclic groups,

cyclic groups, aryl groups or hydrogen, and wherein the R-designated groups in addition can contain one or more functional groups selected from the group consisting of hydroxyl, ether, carboxyl, carbonyl, amine, nitro or halogen;

c) the primary, secondary or tertiary amide promoters contain one or more amide containing groups of the general formula

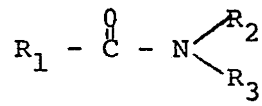

wherein $R_1$, $R_2$, and $R_3$ are, independently, hydrogen, linear or branched acyclic groups, alicyclic groups, cyclic groups or aryl groups and wherein the R-designated groups can in addition contain one or more functional groups selected from the group consisting of hydroxyl, ether, carboxyl, carbonyl, amine, nitro or halogen;

d) the primary, secondary or tertiary amine promoters contain one or more amine containing groups of the general formula

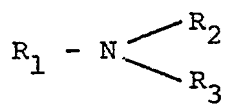

wherein $R_1$, $R_2$, and $R_3$ are, independently, hydrogen, linear or branched acyclic groups, alicyclic groups, cyclic groups, or aryl groups, and wherein the R-designated groups can in addition contain one or more functional groups selected from the group consisting of hydroxyl, ether, carbonyl, halogen, carboxyl, nitro, or amide;

e) the organic acid promoters contain one or more adjacent or non-adjacent carboxylic acid containing group of the general formula

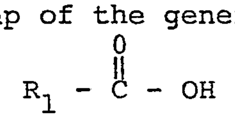

wherein $R_1$ is a hydrogen, linear or branched acyclic group, alicylic group, cyclic group, or aryl group and wherein R can in addition, contain

one or more functional groups selected from the group consisting of carbonyl, hydroxyl, halogen, ether, nitro, amine, or amide;

f) the phenol promoters have one or more functional groups of the general formula

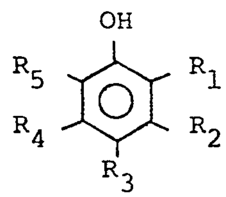

wherein $R_1$, $R_2$, $R_3$, $R_4$, and $R_5$ are, independently, hydrogen, halogen, hydroxyl, nitro, ether, or carbonyl, linear or branched acyclic groups, alicyclic groups, cyclic groups, aryl groups or substituted aryl groups wherein in addition the R-designated groups can contain one or more functional groups selected from the group consisting of halogen, ether, nitro, carboxyl, carbonyl, amine, amide, or hydroxyl, the method comprising heating a metal component with one or more promoters at a temperature of up to 175°C for a period of time of up to two hours and recovering the preformed catalyst.

14. A method as described in claim 13 wherein the metal compound is contacted with the promoter in the presence of a solvent.

15. A method as described in claim 2 wherein the promoter is an alcohol having a pKa of less than 17.

16. A method as described in claim 15 wherein the alcohol is selected from the group consisting of triphenylmethanol, trichloroethanol, trifluoroethanol, 2-nitroethanol, 2-chloroethanol, 2,2-dichloroethanol, 2-methoxyethanol, 2-chlorocyclohexanol, and ortho-chlorobenzyl alcohol.

1/4

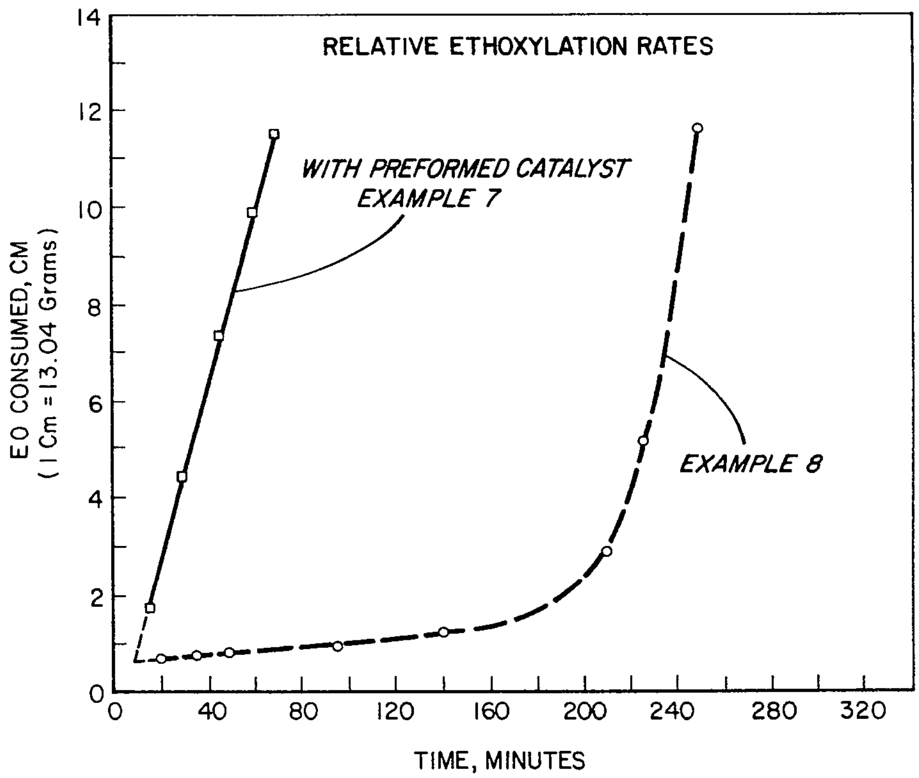
RELATIVE ETHOXYLATION RATES
WITH PREFORMED CATALYST
EXAMPLE 7
EXAMPLE 8
EO CONSUMED, CM
(1 Cm = 13.04 Grams)
0
2
4
6
8
10
12
14
0
40
80
120
160
200
240
280
320
TIME, MINUTES

Fig. 1

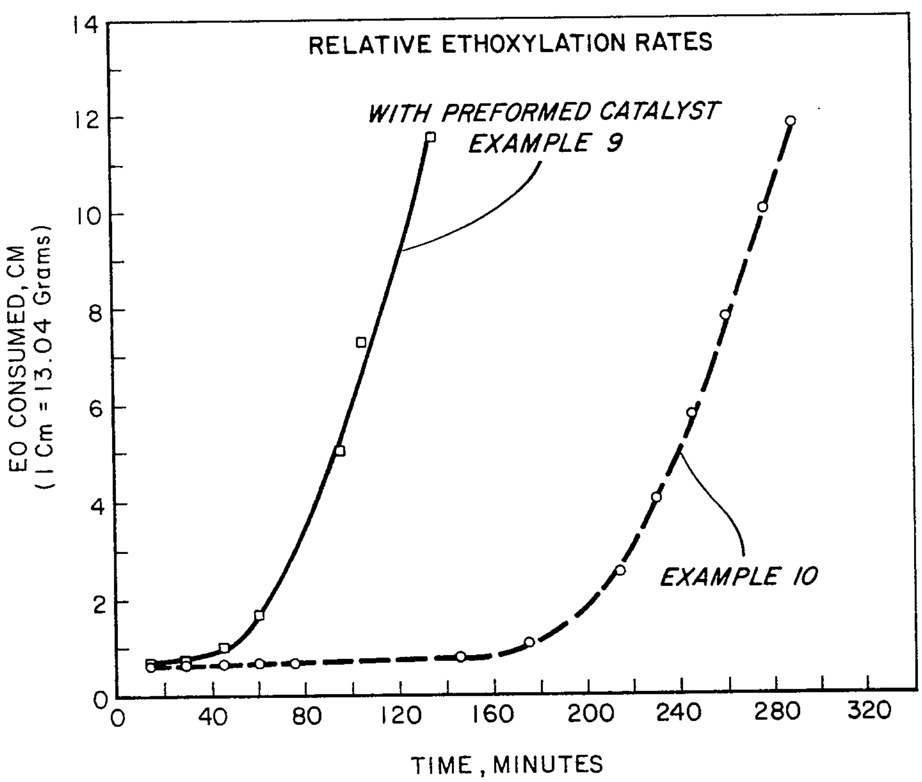
RELATIVE ETHOXYLATION RATES
WITH PREFORMED CATALYST
EXAMPLE 9
EXAMPLE 10
EO CONSUMED, CM
( 1 Cm = 13.04 Grams)
TIME, MINUTES
0
2
4
6
8
10
12
14
0
40
80
120
160
200
240
280
320

Fig. 2

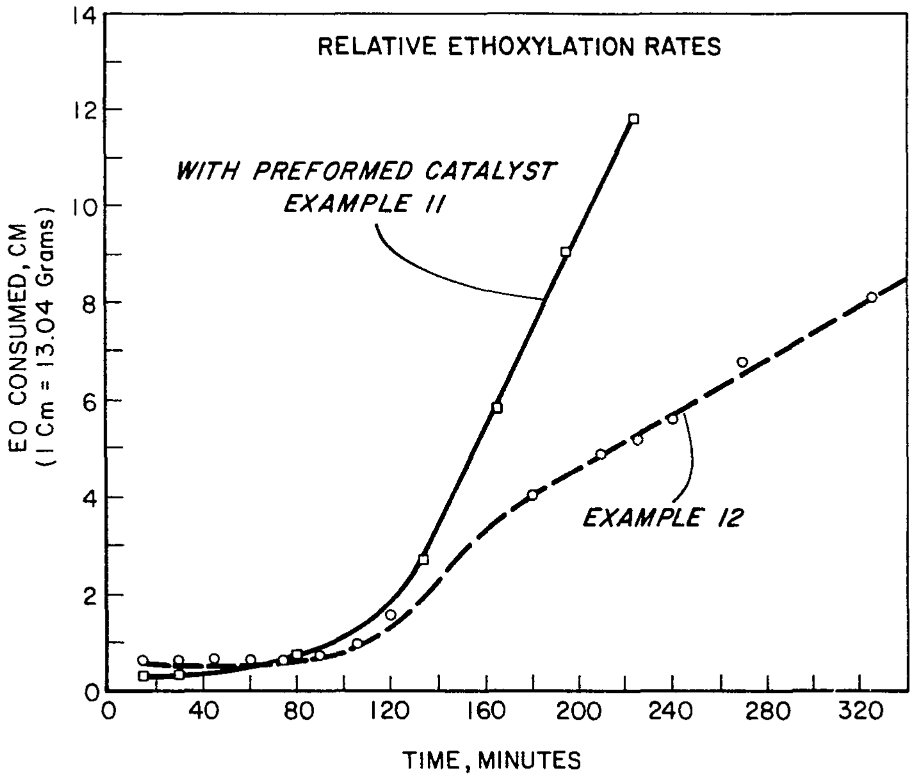
RELATIVE ETHOXYLATION RATES
WITH PREFORMED CATALYST
EXAMPLE 11
EXAMPLE 12
EO CONSUMED, CM
(1 Cm = 13.04 Grams)
TIME, MINUTES
0
2
4
6
8
10
12
14
40
80
120
160
200
240
280
320

*Fig. 3*

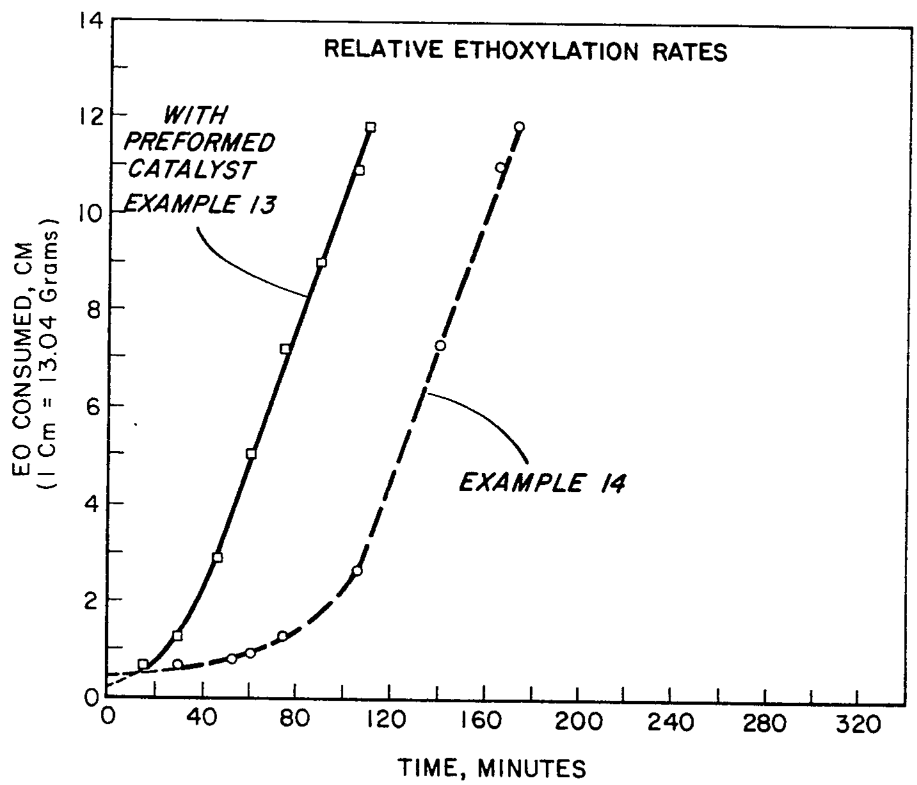
RELATIVE ETHOXYLATION RATES
WITH PREFORMED CATALYST EXAMPLE 13
EXAMPLE 14
EO CONSUMED, CM (1 Cm = 13.04 Grams)
TIME, MINUTES
0
2
4
6
8
10
12
14
0
40
80
120
160
200
240
280
320

Fig. 4

0033760

European Patent Office

# EUROPEAN SEARCH REPORT

Application number

EP 80 10 6315.7

| DOCUMENTS CONSIDERED TO BE RELEVANT | | | CLASSIFICATION OF THE APPLICATION (Int. Cl.3) |
|---|---|---|---|
| Category | Citation of document with indication, where appropriate, of relevant passages | Relevant to claim | |
| E | EP - A1 - 0 020 867 (CONOCO)<br>* claim 1 *<br>-- | 1 | C 07 C 41/03<br>B 01 J 23/02<br>B 01 J 31/00 |
| E | EP - A2 - 0 018 463 (CONOCO)<br>* claim 1 *<br>---- | 1 | |
| | | | TECHNICAL FIELDS SEARCHED (Int. Cl.3) |
| | | | B 01 J 23/02<br>B 01 J 31/00<br>B 01 J 31/02<br>C 07 C 41/01<br>C 07 C 41/03 |
| | | | CATEGORY OF CITED DOCUMENTS<br>X: particularly relevant<br>A: technological background<br>O: non-written disclosure<br>P: intermediate document<br>T: theory or principle underlying the invention<br>E: conflicting application<br>D: document cited in the application<br>L: citation for other reasons |
| X | The present search report has been drawn up for all claims | | &: member of the same patent family, corresponding document |

| Place of search | Date of completion of the search | Examiner |
|---|---|---|
| Berlin | 15-05-1981 | KNAACK |

EPO Form 1503.1 06.78